# EUROPEAN PATENT SPECIFICATION

(11) **EP 4 471 000 B1**
(45) Date of publication and mention of the grant of the patent: **31.12.2025**
(21) Application number: 23211836.4
(22) Date of filing: 23.11.2023
(51) Int. Cl.: C07C 5/333, C10G 3/00, C10G 25/05, C10G 11/00, C10G 53/08, C10G 55/06, C10G 29/20, C10G 45/32, C10G 69/12, C10G 25/12, C10G 55/04, C10G 69/04, C10G 69/06

(54) **PROCESS FOR DEHYDROGENATION OF NORMAL PARAFFINS TO OLEFINS**
VERFAHREN ZUR DEHYDRIERUNG VON NORMALPARAFFINEN ZU OLEFINEN
PROCÉDÉ DE DÉSHYDROGÉNATION DE PARAFFINES NORMALES EN OLÉFINES

(30) Priority: 30.05.2023 US 202363504884 P
(43) Date of publication of application: 04.12.2024
(73) Proprietor: UOP LLC, Rosemont, Illinois 60018 (US)
(72) Inventor: KOLEV, Evgeny T., Charlotte 28202 (US); DO, Phuong T.M., Charlotte 28202 (US)
(74) Representative: Boult Wade Tennant LLP

(56) References cited:
- WO-A2-2008/013519
- US-A1- 2009 158 637
- US-A1- 2013 253 243

## Description

### BACKGROUND

Linear alkylbenzenes are organic compounds with the formula C₆H₅CₙH₂ₙ₊₁. While the alkyl carbon number, "n" can have any practical value, detergent manufacturers desire that alkylbenzenes have alkyl carbon number in the range of 9 to 16 and preferably in the range of 9 to 14. These specific ranges are often required when the alkylbenzenes are used as intermediates in the production of surfactants for detergents. The alkyl carbon number in the range of 9 to 14 falls in line with the specifications of the detergents industry.

Because the surfactants created from alkylbenzenes are biodegradable, the production of alkylbenzenes has grown rapidly since their initial uses in detergent production in the 1960s. The linearity of the paraffin chain in the alkylbenzenes is key to the material's biodegradability and effectiveness as a detergent. A major factor in the final linearity of the alkylbenzenes is the linearity of the paraffin component.

While detergents made utilizing alkylbenzene-based surfactants are biodegradable, previous processes for creating alkylbenzenes are not based on renewable sources. Specifically, alkylbenzenes are currently produced from kerosene refined from crude extracted from the earth. Due to the growing environmental prejudice against fossil fuel extraction and economic concerns over exhausting fossil fuel deposits, there may be support for using an alternate source for biodegradable surfactants in detergents and in other industries.

The C9 to C14 paraffins generated from feedstocks based on vegetable oils or animal fats contain contaminants that can poison dehydrogenation catalysts. The contaminants can also cause discoloration of the linear alkylbenzenes and linear alkylbenzene sulfonates. The contaminants can include aromatics, light oxygenates, fatty acids, fatty esters, and the like. These contaminants need to be removed before the C9 to C14 paraffins are dehydrogenated.

Accordingly, it is desirable to provide decontaminated C9 to C14 paraffins from renewable easily processed triglycerides and fatty acids from vegetable, animal, nut, and/or seed oils to the dehydrogenation unit. Palm kernel oil, coconut oil and babassu oil have a composition that is high in the desirable range of C9-C14 n-paraffins that aligns with the alkyl carbon number range desired of the detergent industry. Such renewable sources also have a high amount of nC16 to nC18 feeds, and it is desirable to convert those feeds to nC9 to nC14 feeds with a high per-pass yield. These nC9 to nC14 intermediate products are useful in eventually making linear alkylbenzene types of detergents through additional process steps. It is further desirable that the resulting nC9 to nC14 paraffins are linear products with a minimum of branched isomer products.
WO 2008/013519 A2 relates to a process for the removal of oxygenates from a paraffin stream.
US 2013/253243 A1 relates to methods for producing linear paraffins and olefins from natural oils. US 2009/158637 A1 relates to the production of aviation fuel from biorenewable feedstocks.

### BRIEF DESCRIPTION OF THE DRAWINGS

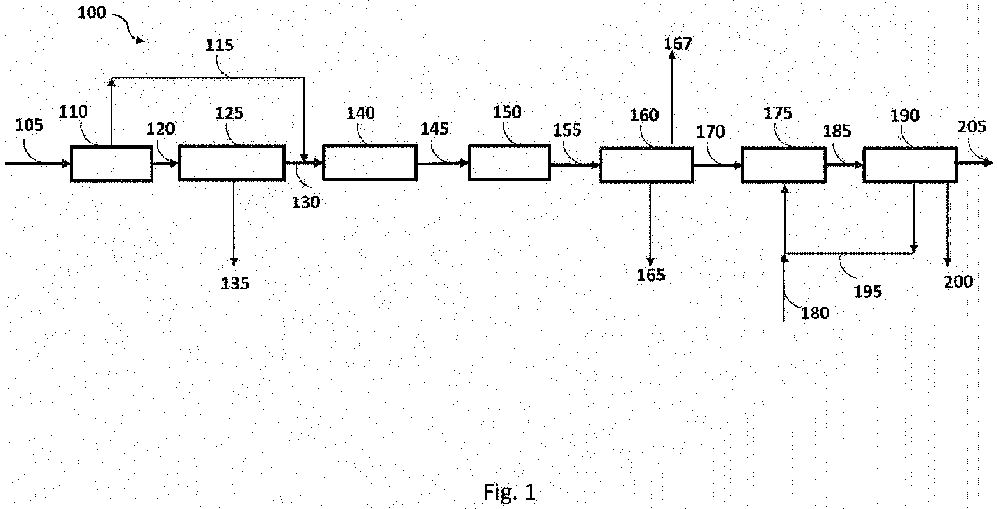

FIG. 1 is a schematic view of one embodiment of a process for producing alkylbenzenes from triglycerides according to the present invention.
Fig. 2 is a plot of the mass-% normal paraffins versus deoxygenation temperature in accordance with Example 2.

### DETAILED DESCRIPTION

The present invention relates to a process according to claim 1.

Natural oils are not based on kerosene or other fossil fuels. Natural oils include those derived from plant or algal material or animal fats, nut, and/or seed oils, and triglyceride-containing oils, and are often referred to as renewable oils. Natural oils typically comprise triglycerides, free fatty acids, or combinations thereof. Natural oils include, but are not limited to, Arachis oil (peanut oil; groundnut oil), Babassu oil, Coconut oil, Cottonseed oil, Grapeseed oil, Maize oil (corn oil), Mustard seed oil, Palm kernel oil, Palm oil, Palm olein (the liquid fraction derived from the fractionation of palm oil), Palm stearin (the high-melting fraction derived from the fractionation of palm oil), Rapeseed oil, Rapeseed oil - low erucic acid (low erucic acid turnip rape oil; low erucic acid colza oil; canola oil), Safflowerseed oil (safflower oil; carthamus oil; kurdee oil), Safflowerseed oil - high oleic acid (high oleic acid safflower oil; high oleic acid carthamus oil; high oleic acid kurdee oil), Sesameseed oil (sesame oil; gingelly oil; benne oil; ben oil; till oil; tillie oil) , Soya bean oil (soybean oil), Sunflowerseed oil (sunflower oil), and Sunflowerseed oil - high oleic acid (high oleic acid sunflower oil).

The feed stream derived from the natural oils comprises normal paraffins, iso paraffins, olefins, oxygenates, and up to 10 wt.% aromatics.

The contaminants in the feed stream include oxygenates and aromatics and may include fatty acids and esters. Alkaline or alkaline earth cation exchange X-Zeolite is used to remove at least a portion of the oxygenates, aromatics, and fatty acids and esters in the paraffin stream derived from the natural oil. Suitable adsorbents for removing the oxygenates and aromatics include, but are not limited to, alkaline or alkaline earth cation exchange X-Zeolite.

Following contaminant removal, the treated stream comprising no more than 6000 ppm of the aromatics and no more than 100 ppm of the oxygenates.

A second adsorbent bed can optionally be included to further reduce the level of oxygenates, aromatics, and fatty acids and esters in the treated stream. The second adsorbent includes, but is not limited to, 5A zeolite.

The first and/or second adsorbent bed can be regenerated at a predetermined time to remove at least a portion of the oxygenates, or aromatics, or both adsorbed onto the first and/or second adsorbent.

There can be one or more first adsorbent beds and one or more second adsorbent beds.

The paraffin stream from renewable feedstocks comprising vegetable oils and animal fats may also contain other contaminants, such as sulfur compounds, or nitrogen compounds, or phosphorous compounds, or combinations thereof. These contaminants may be removed by passing the treated stream through a third adsorbent bed containing a third adsorbent. The third adsorbent may include, but is not limited to, 13X zeolite, 5A zeolite, an alumina-zeolite, or combinations thereof.

After the feed stream derived from natural oils has passed through the first adsorbent bed, and optionally the second and/or the third adsorbent bed, the treated stream is dehydrogenated to convert at least a portion of the paraffins in the treated stream to olefins. The dehydrogenated stream comprises mono-olefins, di-olefins, and aromatics.

The contaminant removal process can be incorporated into a process for making alkylbenzenes from natural oils. The process involves the deoxygenation of the natural oils to form paraffins. The paraffins are separated (by fractionation or distillation, and the like) into a C9 to C14 stream comprising C9 to C14 paraffins and a C14+ stream comprising C14+ paraffins. The C14+ stream is sent to a separate linear selective cracking unit to crack the C14+ paraffins; the cracked paraffins are fractionated into a first stream comprising the C9 to C14 normal and lightly branched paraffins and a second stream comprising isoparaffins. Contaminants, including but not limited to, oxygenates and/or aromatics and/or fatty acids and ester, and/or sulfur compounds, and/or nitrogen compounds, and/or phosphorous compounds, or combinations thereof, are removed from the C9 to C14 stream and the first stream. The decontaminated stream is dehydrogenated to form olefins, di-olefins, and aromatics. The di-olefins are selectively hydrogenated to form additional olefins, and the aromatics are separated and removed forming an aromatics stream comprising the aromatics and a mono-olefin stream comprising the mono-olefins. Benzene is alkylated with the olefins, and the alkylation effluent comprises alkylbenzenes and benzene. The alkylbenzenes are then isolated.

To limit catalyst deactivation, the feed is treated to remove sulfur contamination before hydrodeoxygenation. Otherwise, sulfur accumulates on the catalyst and leads to deactivation. A high temperature hydrogen treatment was shown to recover some of the lost activity. The degree of hydrodeoxygenation can affect the selectivity to each of the normal paraffins in the 9 to 14 carbon range. A large degree of hydrodeoxygenation can bias the hydrodeoxygenated composition largely in favor of normal dodecane and normal decane to the detriment of normal undecane and normal tridecane. A small degree of hydrodeoxygenation can bias the hydrodeoxygenated composition in favor of normal undecane and normal tridecane to the detriment of normal dodecane and normal decane.

The hydrodeoxygenation reactor temperatures are kept low, less than 343°C (650°F) for typical biorenewable feedstocks and less than 304°C (580°F) for feedstocks with higher free fatty acid (FFA) concentration to avoid polymerization of olefins found in FFA. Generally, hydrodeoxygenation reactor pressure of 700 kPa (100 psig) to 21 MPa (3000 psig) are suitable.

The linearity of the alkylbenzene product is mostly dependent on the linearity of the paraffins used to alkylate the benzene. It is a common rule of thumb by those skilled in the art that the linearity of a paraffin feed drops by 5-7 mass % after dehydrogenation and alkylation. Therefore, paraffin with 97 mass % linearity (or alternatively 3 mass % isoparaffin) would result in an alkylbenzene product with linearity around 90-92 mass %. This sets the requirement for paraffin linearity 5-7 mass % higher than the specification for the alkylbenzene product. Typically the linearity of the paraffin product is measured by UOP 621, UOP411, or UOP732 standard test method available from ASTM.
Linear alkylbenzenes may be analyzed using ASTM Standard Test Method D4337.

In the Figure, an exemplary system 100 for producing an alkylbenzene product from a specific triglyceride feed is illustrated.

In the illustrated embodiment, the selected triglyceride feed 105 is delivered to a deoxygenation unit 110 which also receives a hydrogen feed (not shown). In the deoxygenation unit 110, the fatty acids in the selected triglyceride feed 105 are deoxygenated and converted into normal paraffins. Structurally, triglycerides are formed by three, typically different, fatty acid molecules that are bonded together with a glycerol bridge. The glycerol molecule includes three hydroxyl groups (HO--) and each fatty acid molecule has a carboxyl group (COOH). In triglycerides, the hydroxyl groups of the glycerol join the carboxyl groups of the fatty acids to form ester bonds. Therefore, during deoxygenation, the fatty acids are freed from the triglyceride structure and are converted into normal paraffins. The glycerol is converted into propane, and the oxygen in the hydroxyl and carboxyl groups is converted into water, carbon dioxide, or carbon monoxide. The deoxygenation reaction for fatty acids and triglycerides are respectively illustrated as:

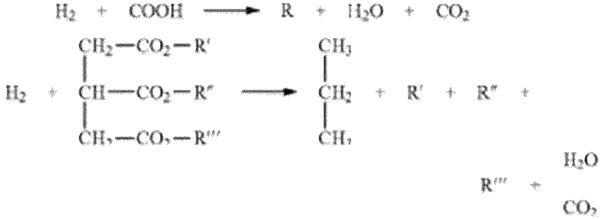

During the deoxygenation reaction, the length of a paraffin chain Rⁿ created will vary by a value of one depending on the exact reaction pathway. It is understood that deoxygenation includes at least one of hydrodeoxygenation, decarboxylation, and decarbonylation, or any combination thereof. For instance, if carbon dioxide is formed, then the chain will have one fewer carbon than the fatty acid source. If water is formed, then the chain will match the length of the fatty acid source.

Operating conditions for the deoxygenating unit include pressures in the range of from 1724 to 5516 kPa (250 to 800 psig) and temperatures in the range of from 274°C to 371°C (525°F to 700°F) in one embodiment, from 274°C to 338°C (525°F to 640°F) in another embodiment and from 274°C to 310°C (525°F to 590°F) in another embodiment. Catalysts may include those containing one or more of Ni, Mo, Co, P, such as Ni--Mo, Ni--Mo--P, NiCo--Mo, or Co--Mo, on aluminas, silica, titania, zirconia, and mixtures thereof. Suitable hydrogen to hydrocarbon mole ratios include from 42.48 to 283.17, from 113.27 to 254.85, and from 141.58 to 226.53 cubic meters per barrel of feedstock (from 1500 to 10,000, from 4000 to 9000, and from 5000-8000 standard cubic feet per barrel of feedstock (scf/B)). Suitable space velocities include 0.2-3.0 hr⁻¹ LHSV. Conditions are selected to minimize cracking or isomerizing the paraffins.

The deoxygenated product containing normal paraffins, water, carbon dioxide, carbon monoxide, and propane is fractionated into a C9 to C14 stream 115 and a C14+ stream 120. The separation may be performed in a multi-stage fractionation unit, distillation system or similar known apparatus. In any event, the separator removes the water, carbon dioxide, carbon monoxide, and propane from the deoxygenated product. A naphtha stream of paraffins with carbon chain lengths of C₅ to C₉ (not shown) may also be formed.

The C14+ stream 120 is sent to the linear selective cracking unit 125 where it is selectively cracked to form a first stream 130 comprising normal or lightly branched C9 to C14 paraffins and a second stream 135 comprising isoparaffins The linear selective cracking takes place in a separate unit, rather than in the bottom bed of a first stage hydrocracking reactor because sulfur and nitrogen contaminants from the first stage can poison a metal-based hydrocracking catalyst. The C14+ paraffins are selectively cracked over the C9 to C14 due to higher absorption energy.

Selection of particular metal catalysts, including noble metals (such as ruthenium and platinum), and nickel can produce a much higher yield of normal paraffins with 9-14 carbons than previous processes. Suitable catalysts include, but are not limited to, Ru/ZrO₂, a Pt-Al₂O₃, Ni-alumina, or a NiOx/clay. With these catalysts, the C14+ stream is able to generate linear cracking products without significant amounts of branched isomer production.

Of the preferred catalysts, the Ru catalyst exhibits much higher activity and per-pass nC9 to nC14 yield than the other catalysts. Under the optimized reaction conditions, it also produces very small amounts of methane and isomerized product. This has been found to be the best catalyst for such chemical transformation process. The Pt-Al2O3 catalyst can produce even lower methane yield than the Ru based catalyst with slightly less linear product yield.

The C9 to C14 stream 115 from the deoxygenation unit 110 and the first stream 130 from the linear selective cracking unit 125 are sent to a decontamination unit 140 as described above. The decontamination unit 140 removes contaminants from the C9 to C14 paraffins in the C9 to C14 stream 115 and the first stream 130. The contaminants include, but are not limited to, oxygenates and/or aromatics and/or fatty acids and ester, and/or sulfur compounds, and/or nitrogen compounds, and/or phosphorous compounds, or combinations thereof.

The decontaminated stream 145 is sent to a dehydrogenation unit 150 where hydrogen is removed to produce a dehydrogenated stream 155 comprising mono-olefins, di-olefins, and aromatics. In the dehydrogenation unit 150, the paraffins are dehydrogenated into mono-olefins of the same carbon numbers as the paraffins. Typically, dehydrogenation occurs through known catalytic processes, such as the commercially popular Pacol process. Di-olefins (i.e., dienes) and aromatics are also produced as an undesired result of the dehydrogenation reactions as expressed in the following equations:
Mono-olefin formation: CₓH₂ₓ₊₂→ CₓH₂ₓ + H₂
Di-olefin formation: CₓH₂ₓ → CₓH₂ₓ₋₂ + H₂
Aromatic formation: CₓH₂ₓ₋₂ → CₓH₂ₓ₋₆ + 2H₂

Operating conditions for the dehydrogenation unit 150 include space velocities from 5 to 50 LHSV and from 20 to 32 LHSV; pressures from 34 kPa (g) to 345 kPa (g) (5 psig to 50 psig) and from 103 kPa (g) to 172 kPa (g) (15 psig to 25 psig); temperatures from 400°C to 500°C and from 440°C to 490°C, and hydrogen to hydrocarbon mole ratios from 1-12 and from 3-7. An example of a suitable catalyst is a Pt on alumina catalyst where platinum is attenuated with an attenuator metal. Another suitable catalyst is described in U.S. Pat. No. 6,177,381. The dehydrogenation unit 150 may be operated dry or with water injection up to 2000 mass-ppm water. Hydrogen can be recycled to the deoxygenation unit upstream.

The dehydrogenated stream 155 is sent to a selective hydrogenation unit 160, such as a DeFine reactor, where at least a portion of the di-olefins are hydrogenated to form additional mono-olefins. As a result, the mono-olefin stream 170 has an increased mono-olefin concentration compared to the dehydrogenated stream 155. The aromatics are separated and removed as aromatics stream 165. A light end stream 167 containing any lights, such as butane, propane, ethane and methane, that resulted from cracking or other reactions during upstream processing can also be removed if needed.

The mono-olefin stream 170 comprising mono-olefins is sent to the alkylation unit 175 along with a benzene stream 180. The benzene is alkylated with the mono-olefins to form alkylbenzene. The alkylation unit 175 contains a catalyst, such as a solid acid catalyst, that supports alkylation of the benzene with the mono-olefins. Fluorinated silica-alumina, hydrogen fluoride (HF), aluminum chloride (AlCl₃), zeolitic, and ionic liquid catalysts are examples of major catalysts in commercial use for the alkylation of benzene with linear mono-olefins and may be used in the alkylation unit 175. As a result of alkylation, alkylbenzene, typically called linear alkylbenzene (LAB), is formed according to the reaction:

C₆H₆ + CₓH₂ₓ → C₆H₅CₓH₂ₓ₊₁

Suitable operating conditions for the alkylation unit 175 include space velocities from 1 to 10 LHSV, pressures to maintain liquid phase operation, such as 2068 kPa (g) to 4137 kPa (g) (300 psig to 600 psig), temperatures in the range of from 80°C to 180°C and 120°C to 170°C, benzene to olefin mole ratios of 3 to 40 and 8 to 35.

Surplus amounts of benzene are supplied to the alkylation unit 175 to achieve high degree of desired alkylation. Therefore, the alkylation effluent 185 exiting the alkylation unit 175 contains alkylbenzene and unreacted benzene. Further the alkylation effluent 185 may also include some unreacted paraffins. The alkylation effluent 185 is passed to a benzene separation unit 190, such as a fractionation column, for separating the unreacted benzene and paraffins from the alkylation effluent 185. The unreacted benzene exits the benzene separation unit 190 in a benzene recycle stream 195 that may be sent back into the alkylation unit 175 to maintain the desired benzene/olefin ratio (e.g., 1-50) to reduce the volume of fresh benzene needed. The fresh benzene requirement (i.e., the net benzene) is determined by the net olefin to the alkylation unit. A paraffin stream 200 can also be separated out and recycled to the dehydrogenation unit 150.

As a result of the post-alkylation separation processes, the linear alkylbenzene product 205 is isolated. It is noted that such separation processes are not necessary in all embodiments in order to isolate the linear alkylbenzene product 205.

The linear alkylbenzene product 205 is a linear alkylbenzene product comprising: alkylbenzenes having the formula C₆H₅CₙH₂ₙ₊₁ wherein n is from 9 to 14. In some embodiments, at least 80 mass % of the alkylbenzenes have linear alkyl groups, or at least 90 mass %.

The linear alkylbenzene may be sulfonated to provide a linear alkylbenzene sulfonate product comprising: alkylbenzene sulfonate compounds having the formula CₙH₂ₙ₊₁C₆H₄SO₃H wherein n is from 10 to 14, or wherein n is from 11 to 13.

The term "column" means a distillation column or columns for separating one or more components of different volatilities. Unless otherwise indicated, each column includes a condenser on an overhead of the column to condense and reflux a portion of an overhead stream back to the top of the column and a reboiler at a bottom of the column to vaporize and send a portion of a bottoms stream back to the bottom of the column. Feeds to the columns may be preheated. The top pressure is the pressure of the overhead vapor at the vapor outlet of the column. The bottom temperature is the liquid bottom outlet temperature. Unless indicated otherwise, overhead lines and bottoms lines refer to the net lines from the column downstream of any reflux or reboil take-off to the column. Stripper columns may omit a reboiler at a bottom of the column and instead provide heating requirements and separation impetus from a fluidized inert media such as steam.

As used herein, the term "a component-rich stream" or "a component stream" means that the stream coming out of a vessel has a greater concentration of the component than the feed to the vessel. As used herein, the term "a component-lean stream" means that the lean stream coming out of a vessel has a smaller concentration of the component than the feed to the vessel.

### EXAMPLES

### Example 1

A coconut oil feed was deoxygenated, to form paraffins, dehydrogenated to form mono-olefins, and benzene was alkylated with the mono-olefins to form an alkylbenzene product with a modern carbon content of 62 mass 96 modern carbon as determined by ASTM D6866 as compared to a theoretical modern carbon content of 66.4 mass %, a bromine number of 1 g Br/per gram sample as determined by UOP standard test method 304, and a linearity of 92 mass %.

### Example 2

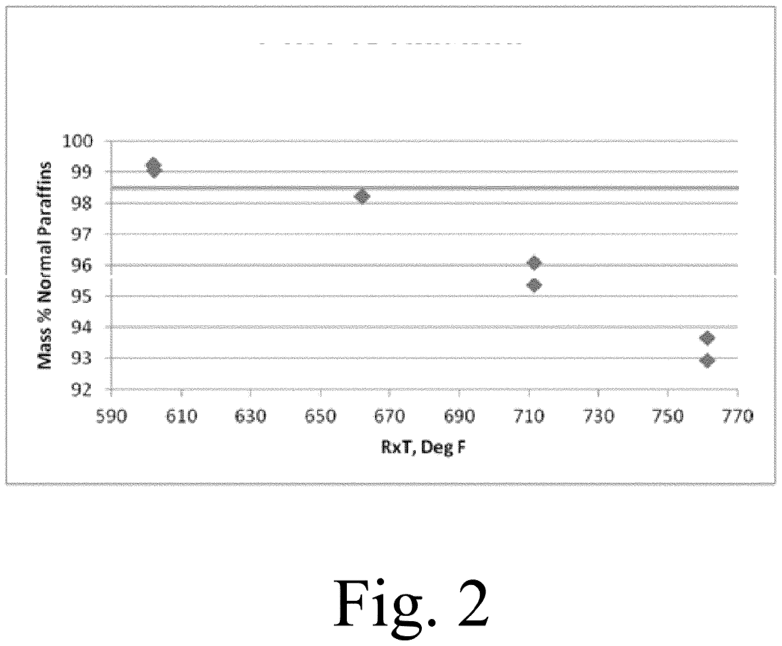

An oil was deoxygenated using a catalyst at a pressure of 3.31 MPa (480 psig_, H, to bio-oil ratio of 203.88 m³/B (7200 scf/B) and a LHSV of 1 hr'. During operation, the deoxygenation reaction temperature was increased in steps from 315°C. (600°F) to 34.9°C. (660°F) and then to 377°C. (710°F) and 404°C. (760°F) to monitor the response of linearity in the final product to reaction temperature. The results are shown in Fig. 2 which is a plot of the concentration in mass % of normal C10-C 13 paraffins versus reaction temperature. Fig. 2 clearly demonstrates that as the deoxygenation reaction temperature is increased, the concentration of linear paraffins decreases. Controlling the temperature to less than 404°C. (760°F) resulted in greater than 92 mass percent linear paraffins.

Note: Examples 1 and 2 were previously included in US Patent No. 9,079,814 as Examples 3 and 4.

In the foregoing, all temperatures are set forth in degrees Celsius and, all parts and percentages are by weight, unless otherwise indicated.

## Claims

1. A method for dehydrogenation of normal paraffins to olefins comprising:
passing a feed stream (130) derived from natural oils comprising C9 to C14 normal paraffins, iso paraffins, olefins, oxygenates, and up to 10 wt.% aromatics through a first adsorbent bed containing a first adsorbent comprising alkaline or alkaline earth cation exchange X-Zeolite wherein the adsorbent removes at least a portion of the oxygenates and aromatics from the paraffin stream by adsorption to form a treated stream (145); and
dehydrogenating the treated stream (145) to convert at least a portion of the treated stream (145) to olefins and provide a dehydrogenated stream (155) comprising mono-olefins, di-olefins, and aromatics;
wherein the method further comprises forming the feed stream by:
deoxygenating a natural oil (105) to form a paraffin stream comprising C9 to C28 paraffins; and
linear selective cracking the paraffin stream in a linear selective cracking unit (125) under linear selective cracking conditions in the presence of a linear selective cracking catalyst to form a first stream (130) comprising normal or lightly branched C9 to C14 paraffins and a second stream (135) comprising isoparaffins, wherein the first stream (130) is the feed stream (130).

2. The method of claim 1 further comprising:
regenerating the adsorbent bed at a predetermined time to remove at least a portion of the oxygenates, or aromatics, or both adsorbed onto the adsorbent.

3. The method of any one of claims 1-2 further comprising;
passing the treated stream (145) though a second adsorbent bed containing a second adsorbent comprising 5A zeolite to remove additional oxygenates and aromatics to form a second treated stream.

4. The method of claim 3 further comprising:
regenerating the second adsorbent bed at a predetermined time to remove at least a portion of the oxygenates, or aromatics, or both adsorbed onto the second adsorbent.

5. The method of any one of claims 3-4 further comprising:
removing contaminants from the treated stream in a third adsorbent bed comprising a third adsorbent to form a decontaminated stream (145) wherein the contaminates comprise sulfur compounds, or nitrogen compounds, or phosphorous compounds, or combinations thereof, wherein the third adsorbent comprises 13X zeolite, 5A zeolite, an alumina-zeolite, or combinations thereof before dehydrogenating the treated stream.

6. The method of any one of claims 1-2 further comprising:
selectively hydrogenating the di-olefins in the dehydrogenated stream (155) to form additional mono-olefins, and separating and removing the aromatics from the mono-olefins to form an aromatics stream (165) comprising the aromatics and a mono-olefins stream (170) comprising the mono-olefins:
alkylating benzene (180) with the mono-olefins under alkylation conditions to provide an alkylation effluent (185) comprising alkylbenzenes and benzene; and
isolating the alkylbenzenes to provide the alkylbenzene product (205) derived from the natural oil.

7. The method of any one of claims 1-2 wherein the treated stream comprises no more than 6000 ppm of the aromatics and no more than 100 ppm of the oxygenates.

## Patentansprüche

1. Verfahren zur Dehydrierung von Normalparaffinen zu Olefinen, umfassend:
Durchleiten eines Zufuhrstroms (130), der von natürlichen Ölen abgeleitet ist, umfassend C9- bis C14-Normalparaffine, Isoparaffine, Olefine, sauerstoffhaltige Verbindungen und bis zu 10 Gew.-% Aromaten, durch ein erstes Adsorptionsmittelbett, das ein erstes Adsorptionsmittel enthält, umfassend Alkali- oder Erdalkali-Kationenaustauscher-X-Zeolith, wobei das Adsorptionsmittel mindestens einen Teil der sauerstoffhaltigen Verbindungen und Aromaten aus dem Paraffinstrom durch Adsorption entfernt, um einen behandelten Strom (145) zu bilden; und
Dehydrieren des behandelten Stroms (145), um mindestens einen Teil des behandelten Stroms (145) in Olefine umzuwandeln und einen dehydrierten Strom (155) bereitzustellen, umfassend Monoolefine, Diolefine und Aromaten;
wobei das Verfahren ferner das Bilden des Zufuhrstroms umfasst durch:
Desoxygenieren eines natürlichen Öls (105), um einen Paraffinstrom zu bilden, umfassend C9- bis C28-Paraffine, und
linear-selektives Cracken des Paraffinstroms in einer Einheit für linear-selektives Cracken (125) unter Bedingungen für linear-selektives Cracken in Gegenwart eines Katalysators zum linear-selektiven Cracken, um einen ersten Strom (130) zu bilden, umfassend normale oder wenig verzweigte C9- bis C14-Paraffine, und einen zweiten Strom (135), umfassend Isoparaffine, wobei der erste Strom (130) der Zufuhrstrom (130) ist.

2. Verfahren nach Anspruch 1, ferner umfassend:
Regenerieren des Adsorptionsmittelbetts zu einem vorgegebenen Zeitpunkt, um mindestens einen Teil der an dem Adsorptionsmittel adsorbierten sauerstoffhaltigen Verbindungen oder Aromaten oder beides zu entfernen.

3. Verfahren nach einem der Ansprüche 1 bis 2, ferner umfassend:
Durchleiten des behandelten Stroms (145) durch ein zweites Adsorptionsmittelbett, das ein zweites Adsorptionsmittel enthält, umfassend 5A-Zeolith, um zusätzlich sauerstoffhaltige Verbindungen und Aromaten zu entfernen, um einen zweiten behandelten Strom zu bilden.

4. Verfahren nach Anspruch 3, ferner umfassend:
Regenerieren des zweiten Adsorptionsmittelbetts zu einem vorgegebenen Zeitpunkt, um mindestens einen Teil der an dem zweiten Adsorptionsmittel adsorbierten sauerstoffhaltigen Verbindungen oder Aromaten oder beides zu entfernen.

5. Verfahren nach einem der Ansprüche 3 bis 4, ferner umfassend:
Entfernen von Verunreinigungen aus dem behandelten Strom in einem dritten Adsorptionsmittelbett, umfassend ein drittes Adsorptionsmittel, um einen entkontaminierten Strom (145) zu bilden, wobei die Verunreinigungen Schwefelverbindungen oder Stickstoffverbindungen oder Phosphorverbindungen oder Kombinationen davon umfassen, wobei das dritte Adsorptionsmittel 13X-Zeolith, 5A-Zeolith, ein Aluminiumoxid-Zeolith oder Kombinationen davon umfasst, bevor der behandelte Strom dehydriert wird.

6. Verfahren nach einem der Ansprüche 1 bis 2, ferner umfassend:
selektives Hydrieren der Diolefine in dem dehydrierten Strom (155), um zusätzliche Monoolefine zu bilden, und Abtrennen und Entfernen der Aromaten aus den Monoolefinen, um einen Aromatenstrom (165) zu bilden, umfassend die Aromaten, und einen Monoolefinstrom (170), umfassend die Monoolefine:
Alkylieren von Benzol (180) mit den Monoolefinen unter Alkylierungsbedingungen, um einen Alkylierungsabstrom (185) bereitzustellen, umfassend Alkylbenzole und Benzol; und
Isolieren der Alkylbenzole, um das von dem natürlichen Öl abgeleitete Alkylbenzolprodukt (205) bereitzustellen.

7. Verfahren nach einem der Ansprüche 1 bis 2, wobei der behandelte Strom nicht mehr als 6000 ppm der Aromaten und nicht mehr als 100 ppm der sauerstoffhaltigen Verbindungen umfasst.

## Revendications

1. Procédé de déshydrogénation des paraffines normales en oléfines comprenant :
le passage d'un flux d'alimentation (130) dérivé d'huiles naturelles comprenant des paraffines normales en C9 à C14, des isoparaffines, des oléfines, des composés oxygénés et jusqu'à 10 % en poids d'aromatiques à travers un premier lit d'adsorbant contenant un premier adsorbant comprenant un X-Zeolite échangeur de cations alcalin ou alcalino-terreux, dans lequel l'adsorbant élimine au moins une partie des composés oxygénés et des aromatiques du flux de paraffine par adsorption pour former un flux traité (145) ; et
la déshydrogénation du flux traité (145) pour convertir au moins une partie du flux traité (145) en oléfines et fournir un flux déshydrogéné (155) comprenant des mono-oléfines, des di-oléfines et des aromatiques ;
dans lequel le procédé comprend en outre la formation du flux d'alimentation par :
désoxygénation de l'huile naturelle (105) pour former un flux de paraffine comprenant des paraffines C9 à C28 ; et
un craquage sélectif linéaire du flux de paraffine dans une unité (125) de craquage sélectif linéaire dans des conditions de craquage sélectif linéaire en présence d'un catalyseur de craquage sélectif linéaire pour former un premier flux (130) comprenant des paraffines en C9 à C14 normales ou légèrement ramifiées et un deuxième flux (135) comprenant des isoparaffines, dans lequel le premier flux (130) est le flux d'alimentation (130).

2. Procédé de la revendication 1, comprenant en outre :
la régénération du lit d'adsorbant à un moment prédéterminé pour éliminer au moins une partie des composés oxygénés, ou des aromatiques, ou les deux adsorbés sur l'adsorbant.

3. Procédé de l'une quelconque des revendications 1 et 2, comprenant en outre :
le passage du flux traité (145) à travers un deuxième lit d'adsorbant contenant un deuxième adsorbant comprenant une zéolite 5A pour éliminer les composés oxygénés et aromatiques supplémentaires afin de former un deuxième flux traité.

4. Procédé de la revendication 3, comprenant en outre :
la régénération du deuxième lit d'adsorbant à un moment prédéterminé pour éliminer au moins une partie des composés oxygénés, ou des aromatiques, ou les deux adsorbés sur le deuxième adsorbant.

5. Procédé de l'une quelconque des revendications 3 et 4, comprenant en outre :
l'élimination des contaminants du flux traité dans un troisième lit d'adsorbant comprenant un troisième adsorbant pour former un flux décontaminé (145) dans lequel les contaminants comprennent des composés sulfurés, ou des composés azotés, ou des composés phosphoreux, ou des combinaisons de ceux-ci, dans lequel le troisième adsorbant comprend une zéolite 13X, une zéolite 5A, une alumine-zéolite ou des combinaisons de celles-ci avant la déshydrogénation du flux traité.

6. Procédé de l'une quelconque des revendications 1 et 2, comprenant en outre :
l'hydrogénation sélective des di-oléfines dans le flux déshydrogéné (155) pour former des mono-oléfines supplémentaires, et la séparation et l'élimination des aromatiques des mono-oléfines pour former un flux d'aromatiques (165) comprenant les aromatiques et un flux de mono-oléfines (170) comprenant les mono-oléfines :
l'alkylation du benzène (180) avec les mono-oléfines dans des conditions d'alkylation pour fournir un effluent d'alkylation (185) comprenant des alkylbenzènes et du benzène ; et
l'isolation des alkylbenzènes pour fournir le produit alkylbenzène (205) dérivé de l'huile naturelle.

7. Procédé de l'une quelconque des revendications 1 à 2, dans lequel le flux traité ne comprend pas plus de 6 000 ppm d'aromatiques et pas plus de 100 ppm de composés oxygénés.
